# EUROPEAN PATENT APPLICATION

(11) **EP 4 813 999 A1**
(43) Date of publication of application: **30.09.2026**
(21) Application number: 24894554.5
(22) Date of filing: 19.11.2024
(51) Int. Cl.: C07K 5/083, A61K 38/06, A61K 9/00, A61P 27/02

(54) **NOVEL PEPTIDE DERIVATIVE AND PHARMACEUTICAL COMPOSITION FOR PREVENTING OR TREATING MACULAR DEGENERATION COMPRISING SAME**

(30) Priority: 23.11.2023 KR 20230163932
(71) Applicant: Supadelixir Inc., Chuncheon-si, Gangwon-do 24232 (KR)
(72) Inventor: HAHN, Jang-Hee, Chuncheon-si Gangwon-do 24375 (KR); LEE, Jaeseob, Yanggu-gun Gangwon-do 24539 (KR); JU, Hyunmi, Chuncheon-si Gangwon-do 24320 (KR); LEE, Sang Hyeup, Daegu 42271 (KR); RA, Choon Sup, Daegu 42251 (KR)
(74) Representative: González López-Menchero, Álvaro Luis
(86) International application number: PCT/KR2024/018198
(87) International publication number: WO 2025/110662

(57) **Abstract**

The present invention provides: a novel peptide derivative or a pharmaceutically acceptable salt thereof; and a pharmaceutical composition comprising the novel peptide derivative or the pharmaceutically acceptable salt thereof as an active ingredient for the prevention or treatment of macular degeneration.

## Description

### Technical Field

The present disclosure relates to a novel peptide derivative or a pharmaceutically acceptable salt thereof, and to a pharmaceutical composition for preventing or treating macular degeneration, the pharmaceutical composition including the novel peptide derivative or the pharmaceutically acceptable salt thereof as an active ingredient.

### Background Art

Macular degeneration is a medical condition in which central vision becomes blurred or vision is completely lost, caused by damage to the macula of the retina. Macular degeneration occurs primarily in people over the age of 50 and is increasing worldwide. Although the cause of macular degeneration has not yet been clearly identified, known risk factors include age, smoking, hypertension, obesity, genetic predisposition, excessive exposure to ultraviolet rays, and low blood antioxidant levels.

In order to treat or ameliorate macular degeneration, anti-vascular endothelial growth factor (VEGF) antibodies such as aflibercept (Eylea^{™}) have been clinically used. However, treatment using such anti-VEGF antibodies requires repeated invasive injections into the eye (i.e., intravitreal injections). Direct injection methods into the eye have several disadvantages, including limited dosage, significantly reduced patient compliance due to repeated invasive injections, high risks of bleeding, pain, infection, and retinal detachment, and the burden of high treatment costs. Therefore, many patients are unable to undergo repeated intraocular/intravitreal injections, which gradually leads to blindness.

Accordingly, there is a need in the art for the development of a therapeutic agent and/or formulation capable of effectively treating or ameliorating macular degeneration while also avoiding invasive injections.

### Disclosure

### Technical Problem

In order to develop compounds capable of effectively treating or ameliorating macular degeneration, the inventors of the present disclosure conducted extensive research on derivatives based on small peptides. As a result, the inventors found that a specific peptide derivative exhibits excellent therapeutic and ameliorating activities against macular degeneration. In particular, the inventors discovered that the specific peptide derivative may be used in the form of eye drops, thereby fundamentally avoiding invasive intraocular/intravitreal injections.

Accordingly, the present disclosure aims to provide a specific peptide derivative exhibiting excellent therapeutic and ameliorating activities against macular degeneration.

The present disclosure also aims to provide a pharmaceutical composition for preventing or treating macular degeneration, the pharmaceutical composition including the specific peptide derivative as an active ingredient.

### Technical Solution

According to an aspect of the present disclosure, provided is a peptide derivative of Formula 1 below or a pharmaceutically acceptable salt thereof.

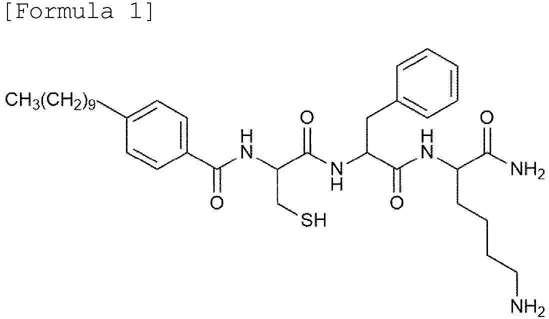

According to another aspect of the present disclosure, provided is a pharmaceutical composition for preventing or treating macular degeneration, the pharmaceutical composition including the peptide derivative of Formula 1 or the pharmaceutically acceptable salt thereof as an active ingredient.

### Advantageous Effects

According to the present disclosure, it has been found that a specific peptide derivative, that is, a peptide derivative of Formula 1 or a pharmaceutically acceptable salt thereof, exhibits excellent therapeutic and ameliorating activities against macular degeneration. In addition, according to the present disclosure, it has been found that the peptide derivative of Formula 1 or the pharmaceutically acceptable salt thereof may be used in the form of eye drops, thereby fundamentally avoiding invasive intraocular/intravitreal injections. Accordingly, a pharmaceutical composition according to the present disclosure may be used effectively for preventing or treating macular degeneration.

### Description of Drawings

FIG. 1 shows evaluation results of cytotoxicity of a peptide (SE101B2L) of the present disclosure in human umbilical vein endothelial cells (HUVECs).
FIG. 2 shows evaluation results of anti-angiogenic activity of a peptide (SE101B2L) of the present disclosure in HUVECs, wherein FIGS. 2a, 2b, and 2c show evaluation results of the effects of the peptide (SE101B2L) of the present disclosure on tube formation, cell migration, and phosphorylation levels of signaling molecules in HUVECs, respectively.
FIG. 3a shows evaluation results of the regulatory ability of a peptide (SE101B2L) of the present disclosure on membrane proteins associated with vascular stability and permeability in HUVECs.
FIG. 3b shows evaluation results of the inhibitory activity of a peptide (SE101B2L) of the present disclosure against permeability caused by changes in membrane proteins associated with vascular stability in HUVECs.
FIG. 4 shows evaluation results of the enhancement activity of a peptide (SE101B2L) of the present disclosure on cell viability reduced by oxidative stress in ARPE19 cells.
FIG. 5 shows evaluation results of the inhibitory activity of a peptide (SE101B2L) of the present disclosure against hydrogen peroxide-induced cellular senescence in ARPE19 cells (FIG. 5a: optical microscopy images; FIG. 5b: quantitative analysis results).
FIG. 6 shows evaluation results of the inhibitory activity of a peptide (SE101B2L) of the present disclosure against hydrogen peroxide-induced apoptosis in ARPE19 cells (FIG. 6a: fluorescence microscopy images; FIG. 6b: quantitative analysis results of apoptotic cells; FIG. 6c: quantitative analysis results of live cells).
FIG. 7 shows evaluation results of the pharmacological activity of a peptide (SE101B2L) of the present disclosure in a macular degeneration animal model (choroidal neovascularization (CNV)-induced chinchilla rabbits), wherein FIGS. 7a and 7b show analysis results of retinal fluorescence intensity on Day 7 and Day 14, respectively.
FIG. 8 shows a spectrum obtained by mass spectrometric analysis of a peptide (SE101B2L) of the present disclosure prepared in Example.

### Best Mode

The present disclosure provides a peptide derivative exhibiting excellent therapeutic and ameliorating activities against macular degeneration, that is, a peptide derivative of Formula 1 below or a pharmaceutically acceptable salt thereof.

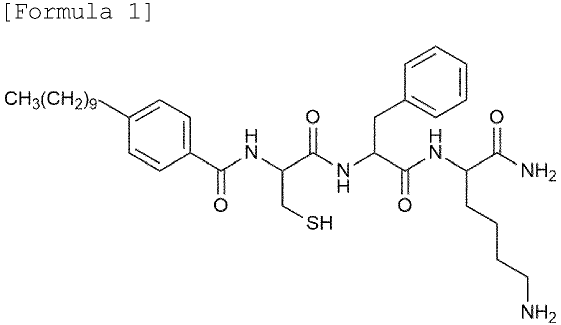

The peptide derivative of Formula 1 may also be denoted as "decylbenzoyl-Cys-Phe-Lys-amide". In the peptide derivative of Formula 1, the amino acids constituting the peptide moiety (i.e., the Cys-Phe-Lys moiety) may each independently be in the form of an L-amino acid or a D-amino acid. The pharmaceutically acceptable salt of the peptide derivative of Formula 1 includes, for example, an acid addition salt, but is not limited thereto.

The peptide derivative of Formula 1 may be prepared by various methods. For example, the peptide moiety (i.e., the Cys-Phe-Lys moiety) may be synthesized by a general Fmoc solid-phase peptide synthesis method (e.g., Adam, G. K.; Patrick, J. S.; Yang, H.; Gretchen, G. Standard Practices for Fmoc-Based Solid-Phase Peptide Synthesis in the Nowick Laboratory (Version 1.7.2), University of California, Irvine, CA, US, 2020), using a reactor (Polypropylene LibraTube, HipepKorea, Korea) and a shaker (EUROSTAR digital, IKA, Germany), or using an automated peptide synthesizer (ASP48S, Peptron, Daejeon, Korea). Introduction of decylbenzoyl at the N-terminus may be performed by coupling 4-n-decylbenzoyl chloride to the N-terminus of the peptide moiety on a solid-phase polymer support in dimethylformamide (DMF) in the presence of diisopropylethylamine (DIEA), or by coupling decylbenzoic acid to the N-terminus of the peptide moiety on a solid-phase polymer support in DMF in the presence of DIEA, using 2-(1H-benzotriazol-1-yl)-1,1,3,3-tetramethyluronium (HBTU) and 1-hydroxybenzotriazole (HOBt) as coupling agents. In this case, when Rink Amide MBHA Resin is used as the solid-phase polymer support, the C-terminus may subsequently be obtained in the form of an amide. The obtained peptide derivative of Formula 1 may be purified and analyzed by reverse-phase high-performance liquid chromatography (reverse-phase HPLC) (Prominence LC-20AB, Shimadzu, Japan), for example, using an Inspire^{™} C18 column (S-5 µm/12 nm. 5 µm (20 x 250 mm), Dikma, USA) or a Vydac Everest C18 column (250 mm x 22 mm, 10 µm). Elution in chromatography may be performed with a water-acetonitrile linear gradient containing 0.1% (v/v) trifluoroacetic acid (5% to 90% (v/v) acetonitrile), and the molecular weight of the obtained product may be confirmed using a mass spectrometer (MS) (AXIMA Assurance^{™}, MALDI-TOF, Shimadzu, Japan), an LC/MS (HP 1100 Series LC/MSD, Hewlett-Packard, Roseville, USA), or the like.

The present disclosure also provides a pharmaceutical composition for preventing or treating macular degeneration, the pharmaceutical composition including the peptide derivative of Formula 1 or the pharmaceutically acceptable salt thereof as an active ingredient.

In the pharmaceutical composition of the present disclosure, the macular degeneration includes, but is not limited to, age-related macular degeneration (AMD), macular dystrophy caused by genetic factors, myopic macular degeneration caused by high myopia, and macular degeneration caused by inflammatory diseases and trauma. AMD is a disease in which the macula degenerates due to aging of the retina. AMD includes both dry (non-exudative) macular degeneration and wet (exudative) macular degeneration. Dry (non-exudative) AMD is associated with drusen, which are cellular debris in the macula that gradually damage light-sensitive cells and lead to vision loss, whereas wet (exudative) AMD involves the growth of blood vessels beneath the macula, causing blood and fluid to leak into the retina. As used herein, the term "macular degeneration" includes all types of macular degeneration mentioned above.

Preferably, the pharmaceutical composition according to the present disclosure may have a formulation of eye drops. The eye drops may be in the form of a solution or a suspension. In one embodiment, the eye drops may include pharmaceutically acceptable additives such as a solubilizing agent, a stabilizing agent, a buffering agent, and a pH-adjusting agent. If necessary, the pharmaceutical composition in the form of eye drops may include a buffering agent such as boric acid, a pH-adjusting agent such as hydrochloric acid or sodium hydroxide, a preservative, and the like, and may be sterilized by a conventional method.

In the pharmaceutical composition according to the present disclosure, the peptide derivative of Formula 1 or the pharmaceutically acceptable salt thereof, which is an active ingredient, may be administered at a concentration of about 0.1 to 200 w/v%, preferably about 1 to 20 w/v%, preferably through instillation. However, the concentration may vary depending on the age, sex, health condition, and severity of disease of a patient. The administration (instillation or the like) may be performed at appropriate intervals. For example, the pharmaceutical composition may be administered once daily or in divided doses according to the instructions of a physician or pharmacist.

Hereinafter, the present disclosure will be described in more detail through examples and test examples. However, these examples and test examples are for illustrating the present disclosure, and the present disclosure is not limited to these examples and test examples.

### Example

A Cys-Phe-Lys peptide was synthesized by an Fmoc solid-phase method using Rink Amide MBHA Resin (385 mg, 0.2 mmol), a reactor (Polypropylene LibraTube^{®}, HipepKorea, Korea), and a shaker (EUROSTAR digital, IKA, Germany).

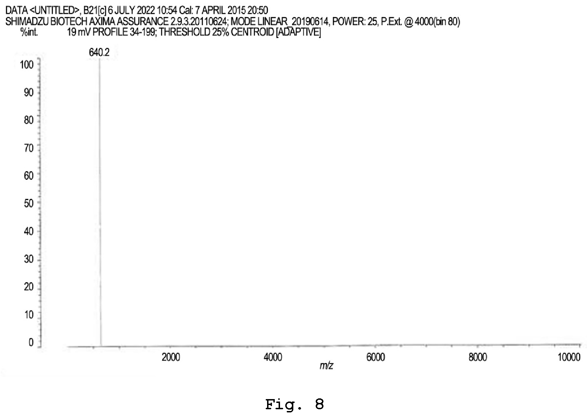

Thereafter, DIEA (0.348 ml) and decylbenzoyl chloride (0.28 ml) were sequentially added to a peptide moiety on a solid-phase polymer support suspended in DMF (8 ml), followed by stirring at room temperature for 1 hour to introduce a decylbenzoyl group at the N-terminus. Subsequently, the peptide derivative having the decylbenzoyl group introduced thereto was released in amide form from the Rink Amide MBHA Resin, and purified and analyzed by reverse-phase HPLC (Prominence LC-20AB, Shimadzu, Japan) using an Inspire^{™} C18 column (S-5 µm/12 nm. 5 µm (20 x 250 mm), Dikma, USA), thereby preparing decylbenzoyl-Cys-Phe-Lys-amide of Formula 1 (hereinafter referred to as "SE101B2L") with a purity of 95% or higher (230 nm). The obtained SE101B2L was identified using an MS (AXIMA Assurance^{™}, MALDI-TOF, Shimadzu, Japan). The results thereof are as shown in FIG. 8 (MS cal.: 639.4, MS observed: [M+H]⁺ = 640.2).

### Test Example 1: Evaluation of Anti-Angiogenic Activity Using HUVECs

The anti-angiogenic activity of the test substance (SE101B2L) was evaluated using HUVECs. HUVECs were cultured in CEFOgro-HUVEC basal medium (CEFO Co., Ltd.) supplemented with growth supplements containing vascular endothelial cell growth-related factors and serum (HUVEC supplements, CEFO Co., Ltd.), which was used as vascular endothelial cell growth medium (hereinafter denoted as "growth medium").

### (1) Cytotoxicity of Test Substance

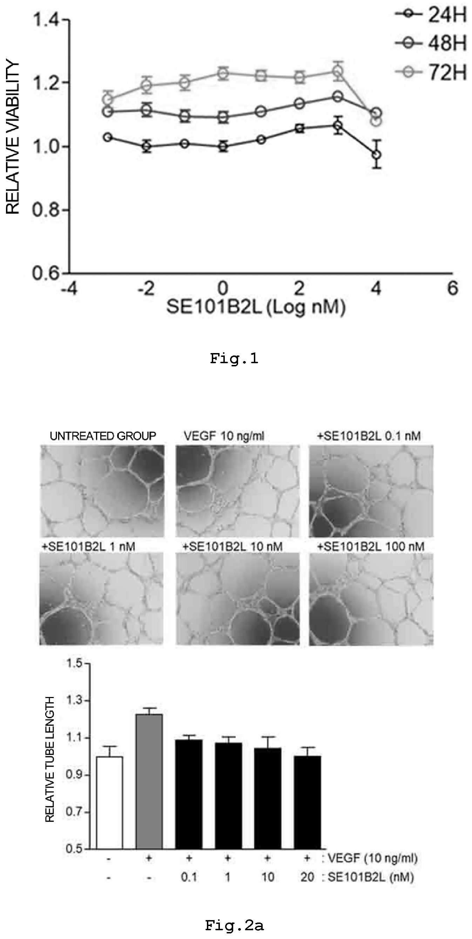

HUVECs were seeded into a 96-well plate at 5 × 10³ cells per well together with 0.1 ml of growth medium. After incubation for one day, the cells were treated with the test substance (SE101B2L) such that the final concentration increased tenfold from 0.001 nM (denoted as log -3) to 10,000 nM (denoted as log 4). Thereafter, test groups were incubated for 24, 48, and 72 hours, and Cell Counting Kit-8 (CCK-8) was added at each predetermined time point, followed by incubation for an additional 2 hours, and absorbance was measured at 450 nm. Cell viability was calculated relative to the absorbance value of the untreated group at 24 hours. The results thereof are as shown in FIG. 1. From the results of FIG. 1, no significant difference in cell viability was observed at each time point up to a concentration of 1,000 nM (1 µM). Accordingly, it is confirmed that the test substance does not exhibit cytotoxicity toward HUVECs up to this concentration.

### (2) Inhibition of Angiogenesis

The experiment was conducted by dividing the groups into an untreated group, a group treated with VEGF alone, and a group co-treated with VEGF and the test substance (SE101B2L).

First, tube formation ability was confirmed to determine whether the test substance inhibited VEGF-induced angiogenesis. HUVECs were seeded into a Matrigel-coated 48-well plate at 5 × 10⁴ cells per well together with 0.5 ml of growth medium. VEGF was added to each VEGF-treated group to a final concentration of 10 ng/ml, while the test substance was added to each test substance-treated group to final concentrations of 0.1, 1, 10, and 100 nM. The test plate was incubated in an incubator with 5% CO₂ at 37°C for 24 hours. After 24 hours, the degree of tube formation was observed under a microscope and photographed. The total tube length in each image was measured using the ImageJ program. The results were converted to values relative to the tube length for the untreated group. The results thereof are as shown in FIG. 2a.

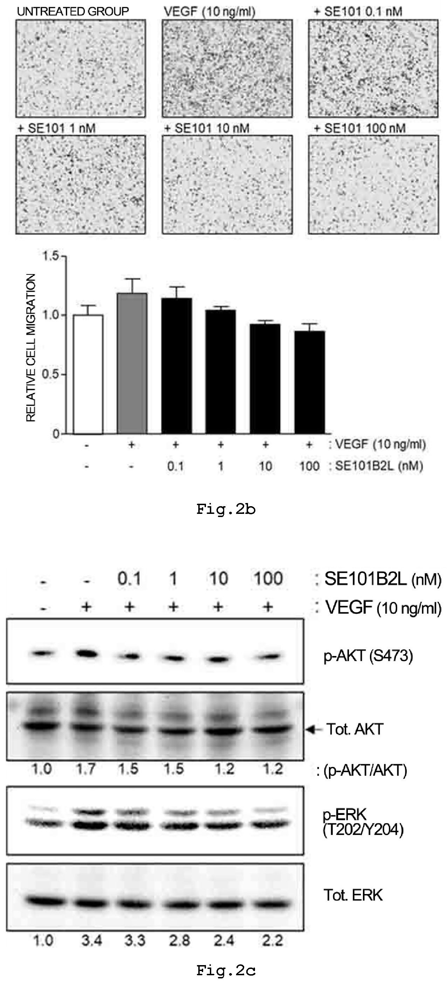

In addition, whether the test substance could inhibit VEGF-induced changes in cell migration ability in HUVECs was confirmed. In terms of cell migration ability, changes in chemotactic cell migration were confirmed using a transwell chamber (24-well plate type). After coating the lower surface of a transwell insert membrane having a diameter of 6.5 mm and a pore size of 8.0 µm with 10 µg of gelatin, 5 × 10⁴ cultured HUVECs were seeded into the inserts using 0.1 ml of HUVEC basal medium. The test substance was added to each test substance-treated group to final concentrations of 0.1, 1, 10, and 100 nM, and VEGF, together with HUVEC basal medium, was added to the lower wells configured in a 24-well plate format to a final concentration of 10 ng/ml, followed by incubation in an incubator with 5% CO₂ at 37°C for 3 hours. Thereafter, the medium was removed from the inserts of the test groups, and the inserts were fixed using a 10% formalin solution. The fixed inserts were then stained with a 0.5% crystal violet solution to stain cells attached to the lower portion of the insert chamber membrane, and unmigrated cells were removed from the inserts using a cotton swab. Migrated cells in each test group were photographed, and the number of cells was counted. The number of counted cells was converted to values relative to that of the untreated group, thereby calculating the results. The results thereof are as shown in FIG. 2b.

Additionally, whether the test substance could regulate the phosphorylation levels of AKT and ERK molecules, which are important in signaling related to VEGF-induced angiogenesis, was tested. Into a 6-well plate containing 2 ml of growth medium, 2 × 10⁵ HUVECs were added and seeded, followed by incubation in an incubator with 5% CO₂ at 37°C for one day. Subsequently, after washing the cells with phosphate-buffered saline (PBS), incubation was performed for an additional 6 hours using CEFOgro-HUVEC medium containing 1% fetal bovine serum. The prepared cells were divided into an untreated group, a group treated with VEGF alone, and groups co-treated with VEGF and the test substance (0.1, 1, 10, and 100 nM), followed by treatment with the test substance. After incubation for an additional 10 minutes, the cells were lysed and collected using a Nonidet P-40 (NP-40) cell lysis buffer containing protease inhibitors including 0.5 mM AEBSF hydrochloride, 150 nM aprotinin, 1 µM E-64, 1 µM leupeptin, and 1 mM PMSF, and phosphatase inhibitors including 50 mM sodium fluoride (NaF) and 1 mM sodium orthovanadate (Na₃VO₄). Protein amounts of the cell lysates were measured by a Bradford assay, followed by separation using sodium dodecyl sulfate-polyacrylamide gel electrophoresis (SDS-PAGE). After transferring the separated protein onto a polyvinylidene fluoride (PVDF) membrane, an immunoassay was performed using antibodies recognizing each target protein. The antibodies used were anti-phospho-AKT (S473) [sc-7985, Santa Cruz Biotech.], anti-phospho-ERK (T202/Y204) [#4370, Cell Signaling Tech.], anti-AKT [sc-271149, Santa Cruz Biotech.], and anti-ERK [sc-135900, Santa Cruz Biotech.]. Each prepared PVDF membrane onto which the proteins had been transferred was blocked for 1 hour using Tris-buffered saline with 0.1% Tween-20 (TBST) containing 3% bovine serum albumin (BSA), followed by a reaction using the antibodies at 4°C for about 16 hours. Then, after removing the remaining antibodies, the membranes were washed with TBST, followed by a reaction at room temperature for 1 hour using antibodies conjugated with horseradish peroxidase (HRP), which recognizes the antibodies. Subsequently, the remaining antibodies were removed in the same manner, followed by a washing process with TBST. ECL solution was then added to the prepared membranes, and the results were confirmed using the Davinch-chemi fluoro imager system. The results thereof are as shown in FIG. 2c.

From the results of FIGS. 2a, 2b, and 2c, it is confirmed that tube formation ability, cell migration ability, and phosphorylation levels of signaling molecules are reduced by the test substance in a concentration-dependent manner. Therefore, it is confirmed that the test substance can inhibit angiogenic activity in macular degeneration by suppressing the VEGF-induced increase in angiogenic ability.

### (3) Confirmation of Changes in Membrane Protein Stability and Permeability of Vascular Endothelial Cells

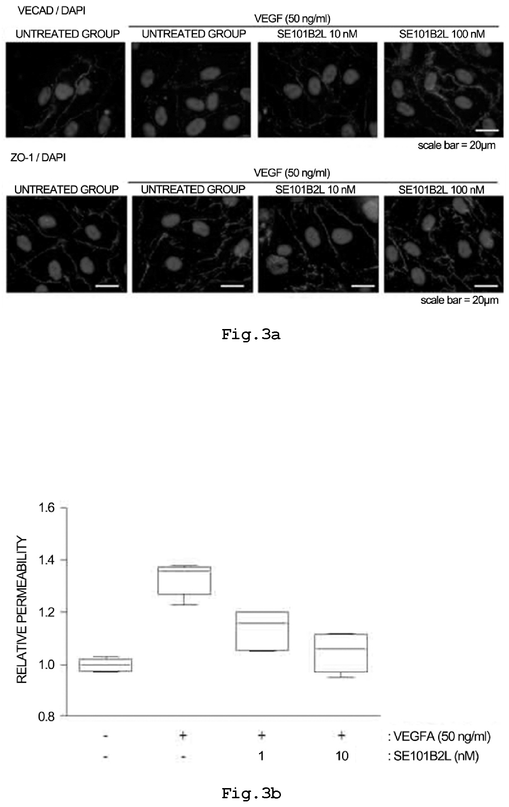

Whether the test substance (SE101B2L) could regulate changes in permeability caused by VEGF-induced changes in membrane protein stability of vascular endothelial cells was confirmed. Expression levels and patterns of VE-cadherin and ZO-1, which are junctional proteins associated with vascular stability and permeability and connect cells to one another, were confirmed. One circular coverslip having a diameter of 12 mm was placed in each well of a 24-well plate, and gelatin was applied using a 2% gelatin solution to facilitate cell attachment. Into the prepared wells, 1 × 10⁵ HUVECs were seeded per well. The cells were cultured in an incubator with 5% CO₂ at 37°C for 4 days, with the growth medium replaced every other day. After confirming that the cells had become completely confluent, the cells were incubated for 1 hour using serum-free CEFOgro-HUVEC medium. At this time, the test substance was added to the corresponding wells to final concentrations of 10 and 100 nM. Upon completion of the incubation, VEGF at a final concentration of 50 ng/mL was added, followed by incubation for an additional 90 minutes. The incubated samples were fixed at room temperature for 15 minutes using a 10% formalin solution. The fixed samples were washed with PBS, followed by permeabilization and blocking processes using PBS (containing 0.2% Triton X-100) containing 3% BSA. Thereafter, a reaction was performed overnight at 4°C using a blocking solution containing an anti-VE-cadherin antibody (#2158, Cell Signaling Tech.) or an anti-ZO-1 antibody (#40-2200, Invitrogen). Subsequently, after washing with PBS, a reaction was performed using a Rhodamine Red-conjugated antibody for 2 hours at room temperature under dark conditions (with external light blocked). Upon completion of the antibody reaction, the samples were washed with PBS and mounted on a slide glass using a mounting solution containing 4',6-diamidino-2-phenylindole (DAPI). The corresponding samples were photographed under a fluorescence microscope. The results thereof are as shown in FIG. 3a.

Additionally, in order to confirm the effect of the test substance on the VEGF-triggered increase in permeability caused by changes in the stability of vascular endothelial membrane proteins, an in vitro endothelial cell permeability assay was performed. The experiment was conducted using a transwell plate of a 24-well plate type including inserts having a diameter of 6.5 mm and a pore size of 0.1 µm. Gelatin was applied using a 2% gelatin solution on cell attachment regions inside the inserts in order to increase cell adhesion. To the lower portions corresponding to the wells of the plate, 1 ml of HUVEC growth medium was added, and 0.3 ml of cell growth medium containing 1 × 10⁵ cells was added to the inserts. The cells were cultured in an incubator with 5% CO₂ at 37°C for 4 days, with the media in the inserts and wells replaced every other day. In order to confirm cell confluency, after transferring the inserts to new empty wells, whether the medium leaked from the inserts into the wells for 5 minutes at room temperature was confirmed. When it was confirmed that no medium leaked out, the media in the inserts and lower wells were replaced with serum-free basal medium, and the test substance was added to the test substance-treated groups to final concentrations of 1 and 10 nM in the inserts and wells. After incubation in an incubator with 5% CO₂ at 37°C for 1 hour, the medium in the wells was replaced with fresh serum-free medium. In the inserts, samples were added to an untreated group, a group treated with VEGF at a final concentration of 50 ng/ml, and groups co-treated with VEGF and the test substance (1 and 10 nM) using serum-free medium containing an HRP-streptavidin conjugate, followed by incubation in an incubator with 5% CO₂ at 37°C for 30 minutes. Subsequently, the inserts were removed, and the serum-free medium was collected from the wells. After dispensing 20 µL of the collected medium sample into each well of a 96-well plate, 50 µL of TMB solution was added to each well to induce a colorimetric reaction, which was then stopped using a 1 M aqueous hydrochloric acid solution. Absorbance was then measured at 450 nm. The measured values were converted to values relative to the absorbance of the untreated group sample. The results thereof are as shown in FIG. 3b.

From the results of FIGS. 3a and 3b, it is confirmed that, with respect to VEGF-induced changes in expression levels and patterns of junctional proteins between cells, treatment with the test substance causes the expression and patterns of the proteins to appear similarly to those of the untreated group in a concentration-dependent manner. In addition, it is confirmed that treatment with the test substance reduces the increase in permeability caused by VEGF-induced reduction in vascular stability. Such results demonstrate that the test substance can inhibit infiltration of substances within blood vessels into ocular tissues caused by uncontrolled angiogenesis occurring in macular degeneration.

### Test Example 2: Evaluation of Inhibitory Activity Against Cellular Senescence and Apoptosis Using Human Retinal Pigment Epithelial Cells

### (1) Cell Culture

Human retinal pigment epithelial cells (ARPE19) were cultured in a humidified incubator with 5% CO₂ at 37°C. DMEM/F12 (Gibco) containing 10% fetal bovine serum and penicillin (100 U/ml)/streptomycin (100 µg/Pml) was used as medium. The cells were cultured with the medium replaced with fresh medium every other day and were passaged using 0.25% trypsin containing 0.02% ethylenediaminetetraacetic acid (EDTA).

### (2) Enhancement of Cell Viability of SE101B2L Under Oxidative Stress

After oxidative stress was induced in human retinal pigment epithelial cells using hydrogen peroxide (H₂O₂), the test substance (SE101B2L) was added at varying concentrations to confirm changes in cell viability. CCK-8 was used to confirm cell viability.

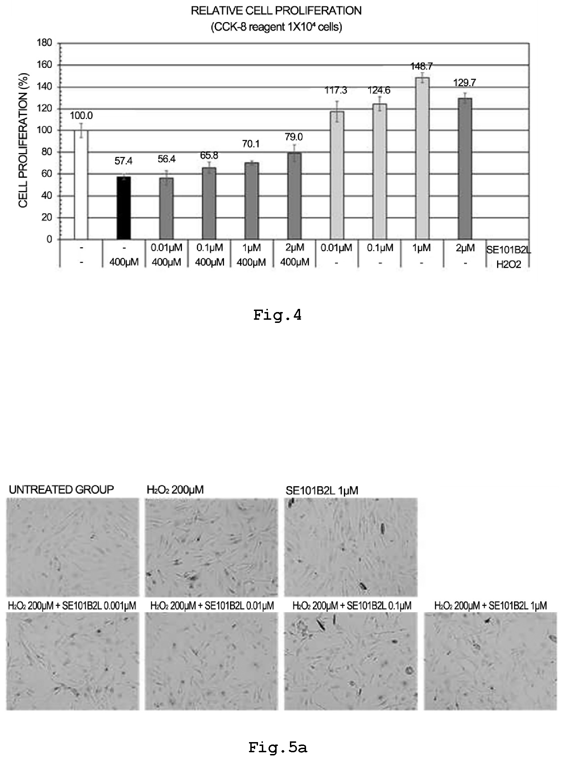

ARPE19 cells were seeded into a 96-well plate at 1 × 10⁴ cells/well and cultured in an incubator for 24 hours. The test substance was added to each treatment group at concentrations of 0, 0.01, 0.1, 1, and 2 µM, together with 400 µM hydrogen peroxide, followed by incubation for 24 hours. In addition, in order to confirm changes upon treatment with the test substance alone, the test substance was added to each group at concentrations of 0.01, 0.1, 1, and 2 µM without treatment with hydrogen peroxide, followed by incubation for 24 hours. The untreated group (denoted as "No treat") was incubated for 24 hours without any treatment. After incubation, a 96-well plate containing 90 µL of cell suspension per well was inoculated with 10 µL of ready-to-use CCK-8 solution. The plate was incubated for 2 hours, and the OD450 of the supernatant was measured to confirm cell viability through quantification. The results thereof are as shown in FIG. 4. From the results of FIG. 4, it is confirmed that cell viability is significantly reduced in the group treated with hydrogen peroxide alone, and that co-treatment with hydrogen peroxide and the test substance increases cell viability in a concentration-dependent manner. Such results demonstrate that the test substance effectively increases cell viability reduced by hydrogen peroxide-induced oxidative stress.

### (3) Inhibition of Hydrogen Peroxide-Induced Cellular Senescence

After cellular senescence caused by oxidative stress was promoted in ARPE19 cells using hydrogen peroxide (H₂O₂), the test substance (SE101B2L) was added at varying concentrations to evaluate inhibitory effects against cellular senescence. Inhibitory activity against cellular senescence was evaluated using senescence-associated β-galactosidase (SA-β-Gal) activity staining, which is well known as a marker for cellular senescence.

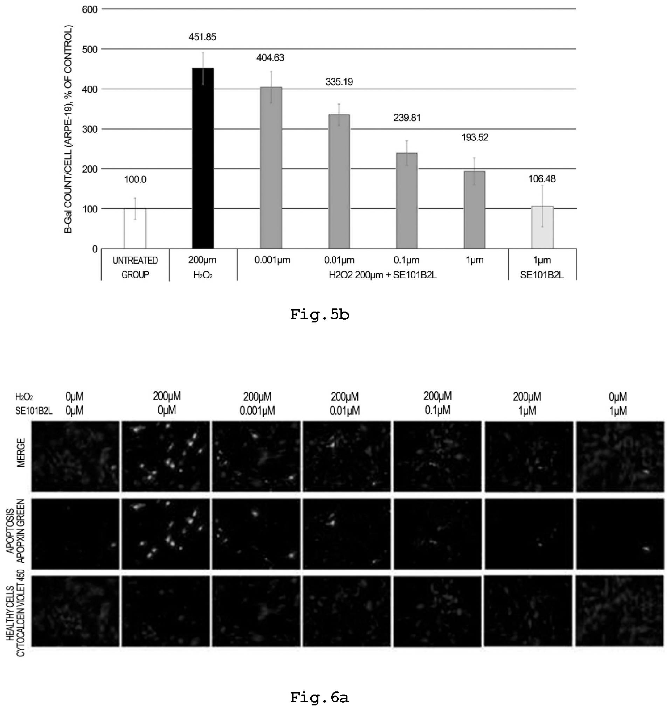

ARPE19 cells were seeded into a 96-well plate at 1 × 10⁵ cells/well and cultured in an incubator with 5% CO₂ at 37°C for 24 hours. When approximately 80% confluency was reached, the test substance was added to each treatment group at concentrations of 0, 0.01, 0.1, and 1 µM, together with 200 µM hydrogen peroxide, followed by incubation for 24 hours. In addition, in order to confirm changes upon treatment with the test substance alone, the test substance was added at a concentration of 1 µM without treatment with hydrogen peroxide, followed by incubation for 24 hours. The untreated group (denoted as "No treat") was incubated for 24 hours without any treatment. After incubation, the cells in each well were washed with PBS and fixed by treatment with 2% formaldehyde for 15 minutes, followed by washing three additional times with PBS. Thereafter, an overnight staining step was performed at 37°C by adding a staining solution using a Senescence β-Galactosidase Staining kit (Cell Signaling Technology) according to the manufacturer's instructions. Cells stained blue were observed under an optical microscope, and the degree of SA-β-gal activity was expressed as a percentage by measuring the number of cells stained blue in the cytoplasm among a total of 50 to 100 cells. The degree of senescence was quantified through measurement. The results thereof are as shown in FIGS. 5a and 5b. From the results of FIGS. 5a and 5b, it is confirmed that co-treatment with hydrogen peroxide and the test substance decreases SA-β-gal activity in a concentration-dependent manner. Such results demonstrate that the test substance effectively inhibits hydrogen peroxide-induced cellular senescence.

### (4) Inhibition of Hydrogen Peroxide-Induced Apoptosis

After apoptosis was induced in ARPE19 cells using hydrogen peroxide (H₂O₂), the test substance (SE101B2L) was added at varying concentrations to evaluate inhibitory effects against apoptosis. Inhibitory effects against apoptosis were measured using phosphatidylserine (PS), which is exposed on the surface of apoptotic cells.

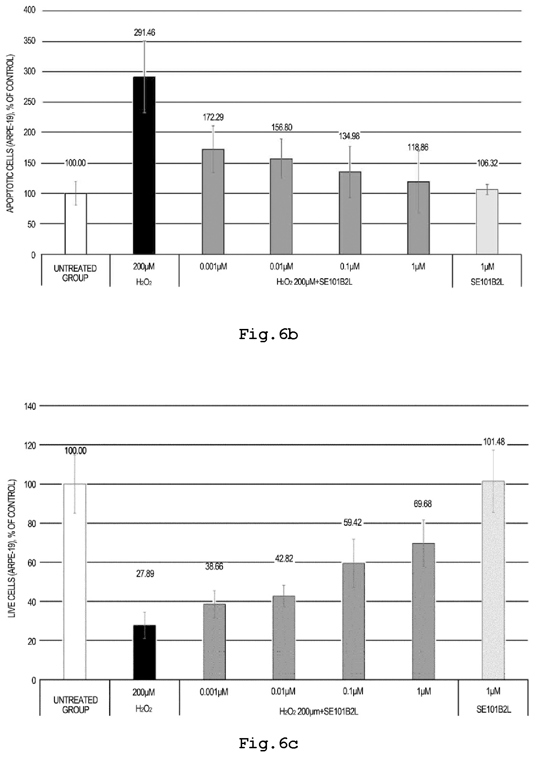

ARPE19 cells were seeded into a 24-well microplate containing coverslips at 1 × 10⁶ cells/well and cultured in an incubator with 5% CO₂ at 37°C for 24 hours. When approximately 80% confluency was reached, the test substance was added to each treatment group at concentrations of 0, 0.01, 0.1, and 1 µM, together with 200 µM hydrogen peroxide, followed by incubation for 24 hours. In addition, in order to confirm changes upon treatment with the test substance alone, the test substance was added at a concentration of 1 µM without treatment with hydrogen peroxide, followed by incubation for 24 hours. The untreated group (denoted as "No treat") was incubated for 24 hours without any treatment. After incubation, for PS measurement, an apoptosis assay kit (Abcam) was used. According to the manufacturer's instructions, washing was performed twice with a washing solution provided by the manufacturer. Without fixing the cells, a staining step was simultaneously performed at room temperature for 1 hour using PS (green) for staining apoptotic cells and CytoCalcein Violet 450 (blue), which is a cytoplasmic marker dye for staining live cells. The cells were fixed by treatment with 2% formaldehyde for 15 minutes and washed three times with PBS, followed by a mounting step. Emission signals detected from each cell were measured using a digital fluorescence microscope (CELENA S digital imaging system, Logos Biosystems, Korea) to quantify apoptotic cells and live cells. The results thereof are as shown in FIGS. 6a, 6b, and 6c. From the results of FIGS. 6a, 6b, and 6c, it is confirmed that hydrogen peroxide causes apoptotic cells (green) to increase and live cells (blue) to decrease, and that, in cells treated with hydrogen peroxide and the test substance, apoptotic cells (green) decrease and live cells (blue) increase in a concentration-dependent manner. Such results demonstrate that the test substance effectively inhibits hydrogen peroxide-induced apoptosis.

### Test Example 3: Evaluation of Pharmacological Activity Against Macular Degeneration

### (1) Test Method

As a macular degeneration animal model, CNV-induced male chinchilla rabbits were used. The efficacy test using the CNV animal model was commissioned to HLB Biostep Co., Ltd. (formerly Notus Co., Ltd.). In order to induce CNV formation, a mydriatic agent (Mydriacyl 1% eye drops) was instilled into the right eye of each chinchilla rabbit, followed by anesthesia. Thereafter, the right eye was irradiated using a laser (LIGHTLas 532, LIGHTMED, USA) under conditions of a wavelength of 532 nm and a power of 150 mW for 0.1 seconds, thereby creating six wounds in the 6 o'clock direction centered on the optic nerve.

Test groups were divided into four groups, as shown in Table 1 below (n = 8). An induced control group (G1) was repeatedly administered 0.9% aqueous sodium chloride solution (vehicle) by instillation twice daily. A positive control group (G2) was administered aflibercept (Eylea^{™}), which is used as a therapeutic agent for macular degeneration, once by intravitreal injection on the day of CNV induction. Test substance-treated groups (G3 and G4) used solutions prepared by dissolving the test substance (SE101B2L) in a vehicle, namely 0.9% aqueous sodium chloride solution, to concentrations of 3750 ppm (G3) and 187.5 ppm (G4), respectively, and were repeatedly administered by instillation twice daily (50 µL per administration). For the administration method, in the case of intravitreal administration, the animals were anesthetized, followed by intravitreal administration into the right eye of each animal using a syringe equipped with a 31-gauge needle. In the case of instillation administration, the test substance was instilled onto the center of the cornea of the right eye using a pipette.

**[Table 1]**

| | Number of animals | Administered substance | Administration route | Administration volume (µL/eye) | Number of administrations |
|---|---|---|---|---|---|
| G1 | 8 | Vehicle | Instillation | 50 | Twice daily |
| G2 | 8 | Eylea^{®} | Intravitreal injection | 50 | Once |
| G3 | 8 | Test substance (3750 ppm) | Instillation | 50 | Twice daily |
| G4 | 8 | Test substance (187.5 ppm) | Instillation | 50 | Twice daily |

After initiation of administration to each group, a mydriatic agent (Mydriacyl 1% eye drops) was instilled into the right eye of each animal on Day 0, Day 7, and Day 14, followed by anesthesia. Approximately 1 ml of a 2% fluorescein sodium salt solution was injected through the ear vein, followed by imaging within about 2 minutes using a fundus camera (TRC-50IX, TOPCON, Japan). Confirmation of retinal CNV and evaluation of pharmacological efficacy were performed using retinal fluorescein fundus images, and image analysis was performed using the ImageJ software (NIH, Bethesda, MD) to analyze fluorescence intensity at irradiated sites.

Assuming normality for the results of the present test, significance between test groups was analyzed using parametric one-way analysis of variance (one-way ANOVA). Statistical analysis was performed using Prism 7.0 (GraphPad Software Inc, San Diego, CA, USA), and a p-value of less than 0.05 was determined to be statistically significant.

### (2) Test Results

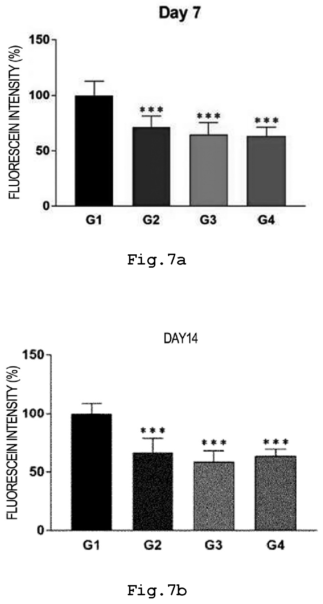

Analysis results of retinal fluorescence intensity on Day 7 and Day 14 after initiation of administration of the test substance are as shown in FIGS. 7a and 7b, respectively. As can be confirmed from the results of FIGS. 7a and 7b, retinal fluorescence intensity levels in the positive control group (G2) and the test substance-treated groups (G3 and G4) were statistically significantly lower than those in the induced control group (G1) (p < 0.001). Such results demonstrate that instillation administration of the peptide derivative according to the present disclosure exhibits pharmacological activity equivalent to that of conventional therapeutic agents for macular degeneration requiring invasive intravitreal injection.

## Claims

1. A peptide derivative of Formula 1 below or a pharmaceutically acceptable salt thereof.

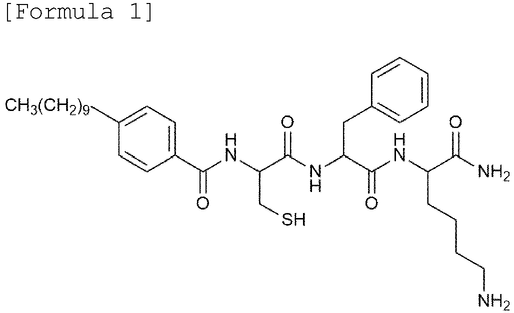

2. A pharmaceutical composition for preventing or treating macular degeneration, the pharmaceutical composition comprising a peptide derivative of Formula 1 below or a pharmaceutically acceptable salt thereof as an active ingredient.

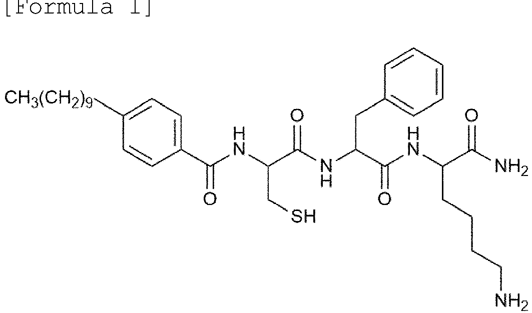

3. The pharmaceutical composition of claim 2, wherein the pharmaceutical composition has a formulation of an eye drop.
